# EUROPEAN PATENT APPLICATION

(11) **EP 3 845 648 A1**
(43) Date of publication of application: **07.07.2021**
(21) Application number: 19853389.5
(22) Date of filing: 03.07.2019
(51) Int. Cl.: C12N 15/115, A61K 31/7088, A61K 51/04, A61P 25/16, A61P 25/28

(54) **BLOOD-BRAIN BARRIER PERMEABLE APTAMER AND APPLICATION THEREOF**

(30) Priority: 30.08.2018 KR 20180102941
(71) Applicant: Nexmos Co., Ltd., Gyeonggi-do 16827 (KR)
(72) Inventor: SON, In Sik, Seongnam-Si Gyeonggi-do 13614 (KR)
(74) Representative: FRKelly
(86) International application number: PCT/KR2019/008148
(87) International publication number: WO 2020/045816

(57) **Abstract**

The present invention relates to an aptamer that penetrating the blood-brain barrier (BBB), and relates to an application of such an aptamer to a complex combined with a substance that selectively binds to a target substance present in the brain. The aptamer and the complex combined with the aptamer can be used as a positron emission tomography (PET) radiographic contrast agent to diagnose Alzheimer's disease or Parkinson's disease at an early stage. It can be used as a radiographic material that can be imaged on, and can be used as a treatment for degenerative brain diseases.

## Description

### Technical Field

The present invention relates to a blood-brain barrier permeable aptamer and application thereof.

### Background Art

In the case of Alzheimer's disease, the aggregation of amyloid beta and TDP-43 between neurons in the brain parenchyma is found, and in Parkinson's disease, an aggregate of proteins called Lewy Body (LBs) in the neurons appears in the pathological findings, and the main component of LB is known as a protein called alpha synuclein. It. It is known that the aggregation of these target substances in the brain occurs before the major symptoms of each disease appear, and such abnormally aggregated proteins cause apoptosis of nerve cells and cause disease. Therefore, it is thought that the incidence of brain diseases can be drastically reduced if the aggregation of target substances related to brain diseases is identified early and the aggregation is prevented.

Aptamer is a single-stranded DNA or RNA (ssDNA or ssRNA), also called "chemical antibody," and refers to a substance that selectively binds to proteins, peptides, etc. by creating a tertiary structure of various shapes through the complementary bonding of bases in the substance. Like an antibody, it has high selective specificity for the target material, but it is as small as 1/10, and it can be produced through synthesis, so it is possible to avoid complex cell culture and purification processes like antibodies, and to maintain uniform biochemical properties. There is an advantage. In addition, DNA or RNA can solve toxicity problems with substances already in the body.

Aptamers can specifically bind to brain disease target substances, and recently, it has been reported that aptamers that specifically bind to a-syn can prevent the aggregation of α-synuclein and promote its decomposition. Therefore, the use of aptamers is expected to be used as a Theragnosis (therapy + diagnosis) technology that can be used for diagnosis and treatment at the same time as it can be used for early diagnosis by binding to a target substance as well as preventing and treating diseases.

However, in order to bind to the target substance present in the brain parenchyma and exhibit efficacy, the aptamer administered through blood must pass through the BBB. The most effective way to deliver substances through the BBB to the brain parenchyma is transcytosis through receptor binding. Receptors known to transfer substances to the brain parenchyma through transcytosis include Transferrin receptor, Insulin receptor, and Low density lipoprotein receptor. In particular, many studies are being conducted to deliver drugs to the brain parenchyma by targeting the Transferrin receptor. For example, it has been reported that an antibody that binds to the Transferrin receptor can effectively cross the BBB. It has also been reported that lysosomal enzymes can be effectively delivered into cells through an aptamer that binds to the extracellular domain (ECD) of the transferrin receptor. However, since these receptors are not only present in the cerebrovascular, drugs delivered to the brain parenchyma targeting the receptor are inevitably delivered to tissues such as the liver, heart, lungs, and kidneys, which may cause unexpected side effects. In order to solve such a problem, research is being conducted to find a substance that specifically passes through blood vessels in the brain. For example, antibodies that selectively pass only BBB through screening using a single-chain antibody library have been reported.

### Prior patent literature

Korean Patent Publication No. 10-2014-0019091

### Disclosure

### Technical Problem

An object of the present invention is to provide an aptamer that passes through the blood-brain barrier.

Another object of the present invention is to provide a novel positron emission tomography (PET) radiographic contrast agent for early diagnosis of Alzheimer's disease or Parkinson's disease.

Another object of the present invention is to provide a novel therapeutic agent for Alzheimer's disease or Parkinson's disease.

### Technical Solution

In order to achieve the above object, the present invention provides an aptamer that passes through the blood-brain barrier (BBB) selected from the oligonucleotide group consisting of the nucleotide sequences set forth in SEQ ID NOs: 1 to 100.

In one embodiment of the present invention, the aptamer preferably has a nucleotide sequence of SEQ ID NO: 101 in common, but is not limited thereto.

In addition, the present invention provides a complex in which a substance selectively binding to a target substance present in the brain and an aptamer passing through the blood-brain barrier (BBB) of the present invention are combined together.

In one embodiment of the present invention, the substance that selectively binds to the target substance present in the brain is a drug, chemical substance, aptamer, siRNA, antisense oligonucleotide, miRNA, CpG oligonucleotide that is difficult to deliver in the brain parenchyma. It is preferably a material selected from the group consisting of creotide, peptide, and protein, but is not limited thereto.

In another embodiment of the present invention, the substance that selectively binds to the target substance present in the brain and the aptamer passing through the Blood-Brain Barrier (BBB) of the present invention are preferably connected by a linker. It is not limited thereto.

In still another embodiment of the present invention, the material that selectively binds to the target material present in the brain is preferably labeled with a radioactive isotope, but is not limited thereto.

In addition, the present invention provides a composition for positron emission tomography (PET) comprising the complex of the present invention as an active ingredient.

In addition, the present invention provides a composition for diagnosing degenerative brain diseases comprising the complex of the present invention as an active ingredient.

In addition, the present invention provides a composition for treating degenerative brain diseases comprising the complex of the present invention as an active ingredient.

In one embodiment of the present invention, the degenerative brain disease is preferably Alzheimer's disease, Parkinson's disease, or Huntington's disease, but is not limited thereto.

The present invention will be described below.

The present invention is a complex in which an aptamer that passes through the blood-brain barrier (BBB) and a substance that selectively binds to a target substance present in the brain are combined together (for example, Bi-specific (dual-specific or chimeric) aptamer) is prepared and used as a positron emission tomography (PET) radiation contrast agent (Aptamer-based radiotracer) to prevent aggregation of the target material to diagnose, prevent and treat Alzheimer's disease or Parkinson's disease at an early stage.

For example, the present invention relates to the development of a material that can be used as the Theragnosis technology to prevent the aggregation of the target material and the distribution of the target material in the brain parenchyma through PET by using the Bi-specific aptamer implemented by connecting with BBB permeable aptamer of the present invention and aptamer selectively binding to various substances that increase aggregation in degenerative brain diseases such as alpha-synuclein, amyloid beta, tau, SOD1, and TDP-43 to inhibit aggregation.

### Effects of the invention

As can be seen through the present invention, the aptamer of the present invention can pass through the blood-brain barrier, and the complex containing the aptamer is used as a positron emission tomography (PET) radiation contrast agent to prevent Alzheimer's disease or Parkinson's disease. It can be diagnosed early, and can be used as a radiographic material that can be used for early imaging of the distribution of the target material in the brain parenchyma through the BBB through PET. In addition, it can be used as a therapeutic agent by preventing aggregation of the target material.

### Description of Drawings

Figure 1 is a picture confirming the expression of ZO-1 in a human blood cerebrovascular cell line (hCMEC/D3),
Figure 2 is a diagram showing the discovery of an aptamer passing through an artificial BBB model by preparing an in vitro BBB model.
Figure 3 is a picture confirming the method and results of the BBB-permeable aptamer using SELEX,
Figure 4 is a picture showing the NGS results of the BBB-permeable aptamer,

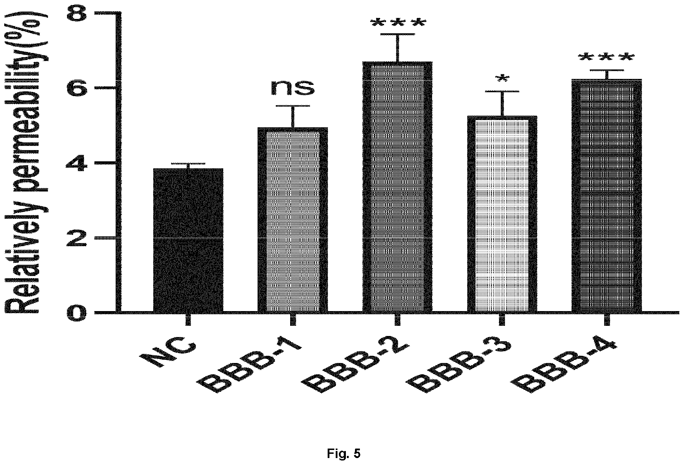

Figure 5 is a diagram showing the results of a transmittance test of BBB-permeable aptamer candidates,

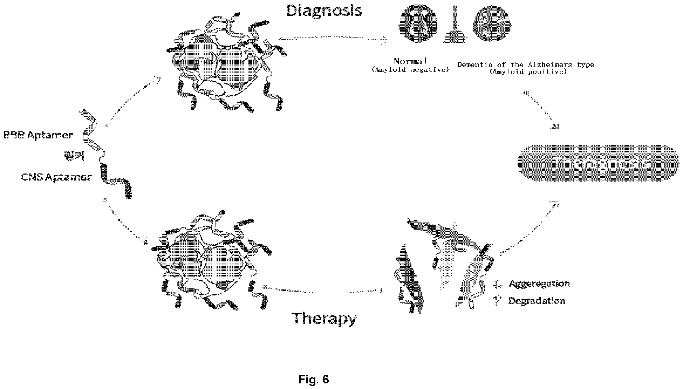

Figure 6 is an example of the application of the present invention, the aptamer of the present invention through the BBB and combined with the aptamer that can bind to the target material present in the brain parenchyma, theragnosis technology utilization strategy through a dual-specific aptamer.

### Mode for Invention

Hereinafter, the embodiments of the present invention will be described in detail with reference to the attached exemplary drawings, as such an example, a person skilled in the art to which the present invention pertains may be implemented in various different forms, it is not limited to the embodiment described here.

### Example 1: Confirmation whether aptamer prevents aggregation or promotes degradation of alpha-synuclein expressed in cells

After overexpressing α-synuclein in human neuroblastoma SH-SY5Y cells using lenti viral vector, aptamers that specifically bind α-synuclein monomer/oligomer are treated. Later, the degree of aggregation and decomposition of α-synuclein was confirmed through western blot.

### Example 2: ZO-1 expression of human blood cerebrovascular cell line (hCMEC/D3)

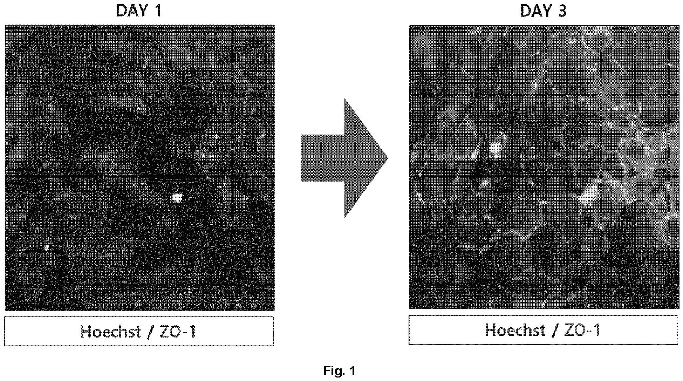

The hCMEC/D3 cell line used to prepare the blood-brain barrier model in vitro is a cell derived from human cerebrovascular cells, mainly the most frequently used for blood-brain barrier studies. Whether or not a phenotypic close junction was formed was confirmed by immunofluorescence staining ZO-1 (green), one of the close junction protein indicators, and the nucleus was stained using Hoechst (blue). As a result, it was confirmed that the longer the culture period was, the better the close junction was formed while the cells were in contact with each other (FIG. 1).

### Example 3: By preparing an in vitro artificial BBB model, aptamer selection is performed to discover and characterize aptamers that specifically penetrates only cerebrovascular

A BBB model was produced in vitro using the human cerebrovascular cell line hCMEC/D3 and hCMEC/D3 cells were purchased from Millipore. The culture medium was EBM-2 (Lonza) supplemented with 5% FBS (gibco), 1.4 µM Hydrocortisone (sigma), 5 µg/ml Ascorbic Acid (sigma), 1 ng/ml bFGF (sigma), 10 mM HEPES (gibco), 1x chemically defined lipid (gibco), P/S (gibco). The exchange of culture medium was performed every 2-3 days. To make a BBB model in vitro, hCMEC/D3 cells were seeded into a transwell (12 well insert, pore size: 0.4µm, corning) with 2.2 x 10⁵ cells and cultured for 6 to 10 days. The DNA aptamer library was treated in the blood vessel of the artificial model using the BBB model, and then the DNA pool was selected (One BBB model was used for each round). After the selection, the aptamer library was treated in a place corresponding to a blood vessel in an artificial model to select a DNA pool that penetrated through hCMEC/D3 cells.

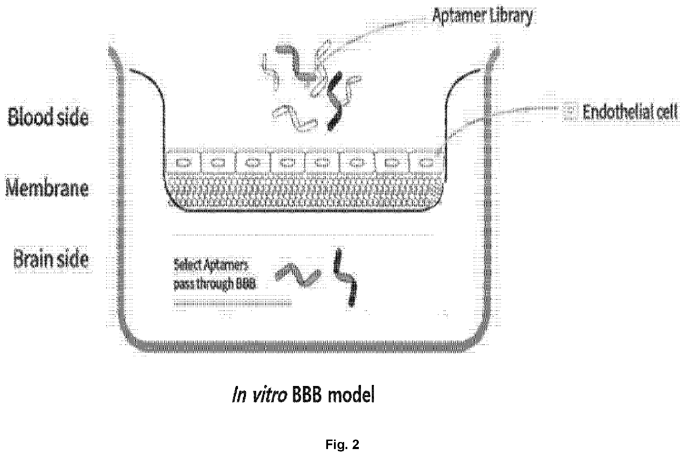

Briefly, as shown in FIG. 2, SELEX was performed to discover an aptamer penetrating through the in vitro blood-brain barrier model. The random DNA library used at this example is a total of 60-mers, and consists of a 30-mer random sequence in the center and a 15-mer fixed primer sequence for amplification at both ends (5'-ATGCGGATCCCGCGC-N30-GCGCGAAGCTTGCGC-3' ;SEQ ID NO: 103). In the first round, 1 nmol of a random DNA library was applied to the blood brain barrier model, reacted at 37°C for 60 minutes, and passed aptamers were selected.

SELEX technology is prepared by folding ssDNA by treating it at 95° C for 10 minutes and leaving it at room temperature for 5 minutes. After washing the cells with PBS, incubate with PBS containing salmon sperm DNA (10 mg/ml) for 10 to 15 minutes to undergo a blocking process. Thereafter, the prepared ssDNA was treated on the blocked cells, and the permeated ssDNA was selected for ssDNA using a phenol/chloroform/isoamyl alcohol (PCI) extraction method. The selected aptamer was amplified by PCR to obtain a DNA sample for the next round.

PCR amplification goes through two steps. At this time, the forward primer was 5'-ATGCGGATCCCGCGC-3' sequence (SEQ ID NO: 104), and the reverse primer was used by attaching biotin to the 5'-GCGCAAGCTTCGCGC-3' (SEQ ID NO: 105) sequence. The first step is to make the selected ssDNA pool into dsDNA using symmetric PCR.

The PCR conditions: Step 1. 95° C 3min Step 2, 95° C 10sec, 59° C 10sec, 72° C 10sec 11∼15 cycle, step3 72° C Imin.

In this step, asymmetric PCR is performed using the created dsDNA as a template with a ratio of the forward and reverse primers to 25:1.

The PCR conditions: Step 1. 95° C 3min Step 2, 95° C 10sec, 59° C 10sec, 72° C 10sec 30cycle, step3 72° C Imin.

Since the ratio of the forward primer is high, more ssDNA is made than dsDNA. At this time, dsDNA contains biotin attached to the reverse primer. In order to separate the ssDNA to be used for SELEX, dsDNA was removed using a magnetic bead to which Streptavidin was attached.

In the second round, the amount of the DNA sample to be treated was reduced to 200 pmol. As the round progressed, the amount of DNA to be treated and the reaction time were reduced to 50 pmol and 20 minutes, respectively. In order to determine whether SELEX was suitable or not, it was monitored in rounds 4 and 9.

It was confirmed by measuring the intensity of the DNA band by electrophoresis using the PCR method.

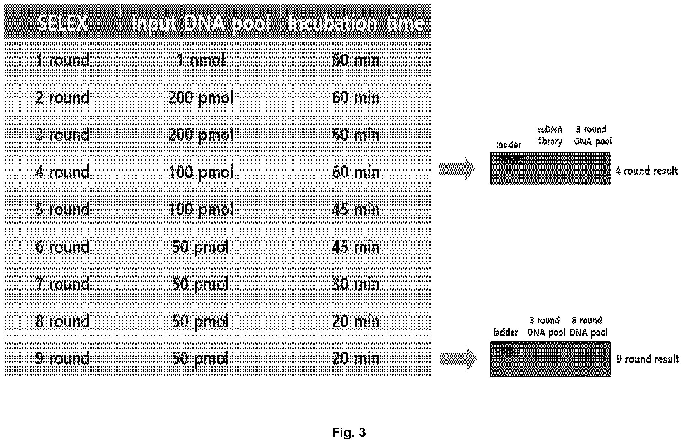

When performing the fourth round, 100 pmol of a random DNA library and a DNA sample selected and amplified in the third round were treated in each blood brain barrier model, and the amount of DNA that penetrated was confirmed through symmetric PCR (PCR amplification same as step 1 condition). As a result, it was confirmed that PCR products were relatively smaller in the group treated with the random DNA library than the DNA samples selected and amplified in the third round. In the ninth round, the DNA samples selected and amplified in the 3rd and 8th rounds were processed in each blood brain barrier model. When the amount of DNA penetrated was confirmed by PCR, it was confirmed that the DNA sample selected in the 8th round penetrated better. Through this, as the round progressed, it was confirmed that an aptamer that better penetrated the blood brain barrier model was selected (FIG. 3). The DNA samples obtained through the 9 rounds were subjected to NGS analysis (for NGS analysis, a sequencer of the illumina platform type was performed by Macrogen).

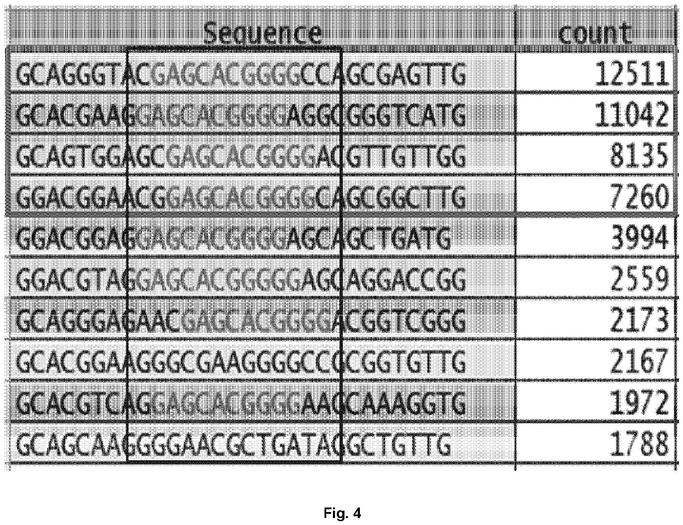

As a result of NGS analysis, it was confirmed that most of the upper sequences contain a common sequence of 10-mer length (GAGCACGGGG; SEQ ID NO: 101), and the common sequence of 10-mer was included in 24 sequences among the upper 100 sequences listed in Table 1 (Figure 4). Experiments were conducted using the top 4 sequences.

### Example 4: permeability test of BBB-permeable aptamer

the 3' part of the 4 aptamer sequences (BBB-1, 2, 3, 4) of the sequence with a high number of reads from the NGS sequence of SEQ ID NOs: 2 to 5 was attached with fluorescent material, Phosphorus FAM and treated on each blood brain barrier model, and the fluorescence intensity of the aptamer passed after 1 hour was measured.

The BBB model used at this example was a smaller model than the BBB model used for SELEX. The model was prepared by culturing 8x10⁴ cells in a 24-well insert transwell (pore size: 0.4um, cornig) for 7 days. 50 pmol of aptamer which each FAM was attached was treated to a part corresponding to the blood vessel part of the model and incubated for 1 hour. After that, the fluorescence intensity of the passed aptamer and the total treated aptamer were measured using Synergy H1 (Bioteck) at emission wavelength of 490 nm and detection wavelength of 520 nm, and the penetration rate was calculated. (Penetration rate = fluorescence intensity of the penetrated aptamer / fluorescence intensity of the treated aptamer X 100)). Compared with the negative control group (5'-CGCGCGTCAGGCATTCCTCACAATTCTTCG-3'; SEQ ID NO: 102), the permeability increased in the entire experimental group, and in particular, BBB-2 and 4 had a large increase, and among them, the penetration rate of BBB-2 was the highest.

### Example 5: Development of bi-Bi-specific (dual-specific or chimeric) aptamer

Bi-specific aptamer is made by linking the BBB permeable aptamer of the present invention and the aptamer for target materials such as α-synuclein or amyloid beta back-to-back through a poly Thymidine linker (T10 ∼ T20). At this example, ribosyUridine or the like is inserted in the linker to be easily broken after transcytosis occurs, so that the aptamer for the target material can be released.

When connected with a linker, the binding affinity of aptamers usually decreases. Therefore, it is necessary to optimize the bi-specific aptamer so that the function of each aptamer remains the same as before connection.

A DNA library is prepared by fixing the sequence of the aptamer penetrating through the BBB, and then SELEX is performed on target materials such as α-synuclein or amyloid beta. A DNA library is prepared by fixing the sequence of an aptamer that binds to a target substance such as α-synuclein or amyloid beta, and then an aptamer that permeates the artificial BBB model is selected.

### Example 6: Confirmation that the bi-specific aptamer can bind to the target material after penetrating through the artificial BBB model

In the artificial BBB model, SH-SY5Y cells overexpressing α-synuclein were cultured on the side corresponding to the brain parenchyma. After treating the bi-specific aptamer labeled with a fluorescent substance on the side corresponding to the blood vessel, it is checked whether fluorescence is visible in the cytoplasm of SH-SY5Y cells. In addition, α-synuclein is fluorescently stained to check how much co-localization is with the aptamer.

**Table 1**

| SEQ ID NO | Sequence |
|---|---|
| 1 | GCAGGGTACGAGCACGGGGCCAGCGAGTTG |
| 2 | GCACGAAGGAGCACGGGGAGGCGGGTCATG |
| 3 | GCAGTGGAGCGAGCACGGGGACGTTGTTGG |
| 4 | GGACGGAACGGAGCACGGGGCAGCGGCTTG |
| 5 | GGACGGAGGAGCACGGGGAGCAGCTGATG |
| 6 | GGACGTAGGAGCACGGGGGAGCAGGACCGG |
| 7 | GCAGGGAGAACGAGCACGGGGACGGTCGGG |
| 8 | GCACGGAAGGGCGAAGGGGCCGCGGTGTTG |
| 9 | GCACGTCAGGAGCACGGGGAAGCAAAGGTG |
| 10 | GCAGCAAGGGGAACGCTGATAGGCTGTTG |
| 11 | GCAGTAAGGGAGGAGCGTGAACTGCTGG |
| 12 | GCAGAAAGGGAGGAGC GAT CAGTG |
| 13 | GGCCAAAGACGGAGGGGACGCTACACTGG |
| 14 | GCAGGAAACGAGCACGGGGGAGCAGGGAGG |
| 15 | GCAGTGAAGACGAGCACGGGGACGAGAATG |
| 16 | GCAGCAAAGGGGGGAGCAGTGTCCATGTTG |
| 17 | GGCCAGCAGGGAGGAGCAGAGCTATACGTG |
| 18 | GGTGGAAGGGTGCGGAGGGGAGGAGCCGTG |
| 19 | GCAGAGTCAACGAGCACGGGGACGTCCATG |
| 20 | GCAGGAAGGGAGGAGCAGGTTGG |
| 21 | GCAGCAGAGGGAGGAGCAGGTGGCCTGTCG |
| 22 | GCAGCATAAGGGAGGAGCAGGGCCACCGTG |
| 23 | GCAGGTACGAGCACGGGGACGGTTGCCCTG |
| 24 | GCAGGGGTGGCCGAGCACGGGGGAGCTGAG |
| 25 | GCAGTTCAACGAGCACGGGGGAGCAGGGTG |
| 26 | GGCACAGCAGAGAAGGGAGACGTGTCCTGG |
| 27 | GCAGCAAGGGGGGAGCGGAACTGGTCATTG |
| 28 | GGACATAAGGGAGCAAGGGGGCCGTGGTTG |
| 29 | GGTGGAGGGGTGCGGGCGGGGGACGTCCTG |
| 30 | GGCAGCAAGGGGGGAGCAGGCCTGAACGTG |
| 31 | GCACGAAGGAGCACGGAGGAGCCGGAATGG |
| 32 | GCAGCAGGGGGAGGAGCAGCTCAGGCCTTG |
| 33 | GCAGTAAGGGAGGAGCGAATTACCCACCGG |
| 34 | GCAGGTAACGAGCACGGGGTCACGGGTATG |
| 35 | GCAGCAGAGGGGGAGCAGGACGACTAGTCG |
| 36 | GCAGGGGACCGAGCACGGGGACGTTCGAGG |
| 37 | GCAGCAAGGGGGGAGCTAGAGCATCTCGTG |
| 38 | GGACGGAGGAGCACGGAGGAGCCGCGAATG |
| 39 | GGCAGCAGAGGGGACGCTGGCCAACTCTCG |
| 40 | GCAGCAAAGGGAGGAGCTGAAGTCACGTTG |
| 41 | GCAGCAGGGAGGAGCTGGTGACCGTACCGG |
| 42 | GCACGAAGGGCAGCAAGGGGGCAGCTGATG |
| 43 | GCAGGAGGGAGGAGCAAGGACATGTCCACG |
| 44 | GGACCGAGGGGAGCACGGGTGGAGCACCTG |
| 45 | GCACGTCAGGAGCACGGGGAAGCAAGGGTG |
| 46 | GCACGAAAGGAGCACGTGGAGGAGCCCGTG |
| 47 | GGCAGGGAGGAGCAACGTTG |
| 48 | GCAGCAAGGGGGAGCAAGAAGGTATCCGTG |
| 49 | GGAGGGGGGGTGCGGCGGGGCCGTGTGTGG |
| 50 | GCAGCAGGGAGGAGCTCAGAGGCCTTTGG |
| 51 | GGACGTCAGGAGCACGGGGACGAGAATAGG |
| 52 | GCAGCAAGGGGACGTAGAAAGGCACTCGTG |
| 53 | GCAGCAAGGGGGGAGCAGAGGACGCTAGTG |
| 54 | GCAGAAGGGAGGAGCAGATGAGACGGTACG |
| 55 | GCAGACAGGGAGACGCAGCTCGATACTCG |
| 56 | GCAGCAAGGGGGAAGCAGTGGAGTACGTTG |
| 57 | GCAGCAAGGGGACGGTGGG |
| 58 | GGCAGCAAGGGGAAGCTCGTG |
| 59 | GCAGCAGGGAGGAGCGGTAATAGCATGTCG |
| 60 | GCACGAAGGAGCACGGGGAAGCCAGCCGTG |
| 61 | GCAGCAAGGGGGACGTCGGATGAACCCTGG |
| 62 | GGCCAAAGCAGCAGGGAGGAGCGATGATTG |
| 63 | GACGAAGGAGCTAGCGGGGGAGCACCCCGG |
| 64 | GCAGCAAGGGGACGTCAGTGAAGCCTCATG |
| 65 | GCAGCAAGGGGGGAGCAGGAGGCATGTGTG |
| 66 | GCAGTAAGGGAGAAGCGTTGGCCTCGTTG |
| 67 | GCAGTGAAACGGAGTAAGGGGCCGGACATG |
| 68 | GCAGTGTTACGAGCACGGGGGAGCCTTATG |
| 69 | GGAGGGGGGGGAGCGGCGGGGGACGTCGTG |
| 70 | GCAGCAAGGGGGACGTAGACTCGTGGTTG |
| 71 | GCAGCAAAGGGGACGCTGATGCAACCCTGG |
| 72 | GGCCAGCAGAGGGGGGAGCAGTGTGACGTG |
| 73 | CCAGCAAGGGAGGAGCAGCTCATGGCCACG |
| 74 | GCACGAAGTGGGAAGGAGGCCGCCCGGTTG |
| 75 | GCAGGTACGAGCACGGGGGAAGCGACTCGG |
| 76 | GCAGCAGAAGAGGAGACGCAGCACCGTGTG |
| 77 | GACGAAGAGAGCGAAGGGGCCGCGGTTGTG |
| 78 | GCAGCAGAAGGGAGGAGCAGTCGAACACTG |
| 79 | GCAGCAAGGGGGAGCGGTGGCCAGTTGTGG |
| 80 | GGCCAAAGCGAAGGGAGACGCGGTTTGTGG |
| 81 | GCAGCAAGGGGGACGTGTGG |
| 82 | GGAGGGAAGGGAGGAGCAGGACTGCCCTGG |
| 83 | GGAGAGCAACGGAGCAGGGGCCGCGGTGTG |
| 84 | GGACGGAGGACAAGCAGGGGGGCCGCCGTG |
| 85 | GCACGAACGGAGCACGGGGACGTCTGTCGG |
| 86 | GCAGCAAGGGGAGGAGCAGCTAACGTGTGG |
| 87 | GCACGAAAGGGAGGAGCTACAGCTGCCATG |
| 88 | GCACGGAGGGAGCAAGGGGCCGCCGGCTGG |
| 89 | CAACGAAGTGGGGAAGGGGCCGCGGACTGG |
| 90 | GCACAAAGGTGGGAGGGGCCGCTTCTCCTG |
| 91 | GCAGCAGGGAGGAGCAAGGATCATCCGTTG |
| 92 | GCAGGGCAACGAGCACGGGGACGACGGAG |
| 93 | GCAGTGATCAAACGAGCACGGGGCCAGTGG |
| 94 | GGAGTGGTGCGAGCACGGGAGGAGCGTACG |
| 95 | GCAGCAAGGGGGACGGGACTGAGTGTATGG |
| 96 | GCAGCAGGGAGGAGCGGAATGGCAATGTTG |
| 97 | GCAGCAAGGGGCCGGGGCGAGACGTAGTTG |
| 98 | GGAGGGAGGAGCGTGTGTTG |
| 99 | GCACGTTAGGGCGGTAGGGGCCGCGGCTGG |
| 100 | GCAGCAAGGGGAACGTGTCG |

Table 1 is a table for the aptamer sequence penetrating the BBB

## Claims

1. An aptamer that penetrating the blood-brain barrier (BBB) selected from the oligonucleotide group consisting of the nucleotide sequences of SEQ ID NOs: 1 to 100.

2. The aptamer of claim 1, wherein the aptamer comprises a nucleotide sequence of SEQ ID NO: 101 in common.

3. A complex in which a substance that selectively binds to a target substance present in the brain and an aptamer penetrates the blood-brain barrier (BBB) of claim 1 or 2 are combined together.

4. The complex of claim 3, wherein the substance selectively binding to the target substance present in the brain is selected from the group consisting of a drug, chemical substance, aptamer, siRNA, antisense oligonucleotide, miRNA, CpG oligonucleotide, peptides and proteins which is difficult to deliver in the brain parenchyma.

5. The complex according to claim 3, wherein the target substance present in the brain is α-synuclein, tau, superoxide dismutase (SOD)1, or amyloid beta.

6. The complex according to claim 3, wherein the substance selectively binding to the target substance present in the brain and the aptamer penetrating the blood-brain barrier (BBB) are connected by a linker.

7. The complex according to claim 3, wherein the substance that selectively binds to the target substance present in the brain is labeled with a radioactive isotope.

8. A composition for positron emission tomography (PET) comprising the complex of any one of claims 3 to 7 as an active ingredient.

9. A composition for diagnosing degenerative brain diseases comprising the complex of any one of claims 3 to 7 as an active ingredient.

10. The composition of claim 9, wherein the degenerative brain disease is Alzheimer's disease, Parkinson's disease, or Huntington's disease.

11. A composition for treating degenerative brain diseases comprising the complex of any one of claims 3 to 7 as an active ingredient.

12. The composition of claim 11, wherein the degenerative brain disease is Alzheimer's disease, Parkinson's disease, or Huntington's disease.


<110>    NEXMOS CO., LTD.

<120>    An aptamer penetrating Blood-Brain Barrier and use of the same

<130>    OP19-0018HSPCT

<150>    10-2018-0102941
<151>    2018-08-30

<160>    105

<170>    KopatentIn 2.0

<210>    1
<211>    30
<212>    DNA
<213>    Artificial Sequence

<220>
<223>    Aptamer


<400>    1
gcagggtacg agcacggggc cagcgagttg                                        30


<210>    2
<211>    30
<212>    DNA
<213>    Artificial Sequence

<220>
<223>    Aptamer


<400>    2
gcacgaagga gcacggggag gcgggtcatg                                        30


<210>    3
<211>    30
<212>    DNA
<213>    Artificial Sequence

<220>
<223>    Aptamer


<400>    3
gcagtggagc gagcacgggg acgttgttgg                                        30


<210>    4
<211>    30
<212>    DNA
<213>    Artificial Sequence

<220>
<223>    Aptamer


<400>    4
ggacggaacg gagcacgggg cagcggcttg                                        30

<210>    5
<211>    29
<212>    DNA
<213>    Artificial Sequence

<220>
<223>    Aptamer


<400>    5
ggacggagga gcacggggag cagctgatg                                          29


<210>    6
<211>    30
<212>    DNA
<213>    Artificial Sequence

<220>
<223>    Aptamer


<400>    6
ggacgtagga gcacggggga gcaggaccgg                                         30


<210>    7
<211>    30
<212>    DNA
<213>    Artificial Sequence

<220>
<223>    Aptamer


<400>    7
gcagggagaa cgagcacggg gacggtcggg                                         30


<210>    8
<211>    30
<212>    DNA
<213>    Artificial Sequence

<220>
<223>    Aptamer


<400>    8
gcacggaagg gcgaaggggc cgcggtgttg                                         30


<210>    9
<211>    30
<212>    DNA
<213>    Artificial Sequence

<220>
<223>    Aptamer

<400>    9
gcacgtcagg agcacgggga agcaaaggtg                                        30


<210>    10
<211>    29
<212>    DNA
<213>    Artificial Sequence

<220>
<223>    Aptamer


<400>    10
gcagcaaggg gaacgctgat aggctgttg                                         29


<210>    11
<211>    28
<212>    DNA
<213>    Artificial Sequence

<220>
<223>    Aptamer


<400>    11
gcagtaaggg aggagcgtga actgctgg                                          28


<210>    12
<211>    24
<212>    DNA
<213>    Artificial Sequence

<220>
<223>    Aptamer


<400>    12
gcagaaaggg aggagcgatc agtg                                              24


<210>    13
<211>    29
<212>    DNA
<213>    Artificial Sequence

<220>
<223>    Aptamer


<400>    13
ggccaaagac ggaggggacg ctacactgg                                         29


<210>    14
<211>    30
<212>    DNA
<213>    Artificial Sequence

<220>
<223>    Aptamer

<400>    14
gcaggaaacg agcacggggg agcagggagg                                        30


<210>    15
<211>    30
<212>    DNA
<213>    Artificial Sequence

<220>
<223>    Aptamer


<400>    15
gcagtgaaga cgagcacggg gacgagaatg                                        30


<210>    16
<211>    30
<212>    DNA
<213>    Artificial Sequence

<220>
<223>    Aptamer


<400>    16
gcagcaaagg ggggagcagt gtccatgttg                                        30


<210>    17
<211>    30
<212>    DNA
<213>    Artificial Sequence

<220>
<223>    Aptamer


<400>    17
ggccagcagg gaggagcaga gctatacgtg                                        30


<210>    18
<211>    30
<212>    DNA
<213>    Artificial Sequence

<220>
<223>    Aptamer


<400>    18
ggtggaaggg tgcggagggg aggagccgtg                                        30


<210>    19
<211>    30
<212>    DNA
<213>    Artificial Sequence

<220>
<223>    Aptamer


<400>    19
gcagagtcaa cgagcacggg gacgtccatg                                        30


<210>    20
<211>    23
<212>    DNA
<213>    Artificial Sequence

<220>
<223>    Aptamer


<400>    20
gcaggaaggg aggagcaggt tgg                                               23


<210>    21
<211>    30
<212>    DNA
<213>    Artificial Sequence

<220>
<223>    Aptamer


<400>    21
gcagcagagg gaggagcagg tggcctgtcg                                        30


<210>    22
<211>    30
<212>    DNA
<213>    Artificial Sequence

<220>
<223>    Aptamer


<400>    22
gcagcataag ggaggagcag ggccaccgtg                                        30


<210>    23
<211>    30
<212>    DNA
<213>    Artificial Sequence

<220>
<223>    Aptamer


<400>    23
gcaggtacga gcacggggac ggttgccctg                                        30


<210>    24
<211>    30
<212>    DNA

<213>    Artificial Sequence

<220>
<223>    Aptamer


<400>    24
gcaggggtgg ccgagcacgg gggagctgag                                        30


<210>    25
<211>    30
<212>    DNA
<213>    Artificial Sequence

<220>
<223>    Aptamer


<400>    25
gcagttcaac gagcacgggg gagcagggtg                                        30


<210>    26
<211>    30
<212>    DNA
<213>    Artificial Sequence

<220>
<223>    Aptamer


<400>    26
ggcacagcag agaagggaga cgtgtcctgg                                        30


<210>    27
<211>    30
<212>    DNA
<213>    Artificial Sequence

<220>
<223>    Aptamer


<400>    27
gcagcaaggg gggagcggaa ctggtcattg                                        30


<210>    28
<211>    30
<212>    DNA
<213>    Artificial Sequence

<220>
<223>    Aptamer


<400>    28
ggacataagg gagcaagggg gccgtggttg                                        30


<210>    29

<211>    30
<212>    DNA
<213>    Artificial Sequence

<220>
<223>    Aptamer


<400>    29
ggtggagggg tgcgggcggg ggacgtcctg                                        30


<210>    30
<211>    30
<212>    DNA
<213>    Artificial Sequence

<220>
<223>    Aptamer


<400>    30
ggcagcaagg ggggagcagg cctgaacgtg                                        30


<210>    31
<211>    30
<212>    DNA
<213>    Artificial Sequence

<220>
<223>    Aptamer


<400>    31
gcacgaagga gcacggagga gccggaatgg                                        30


<210>    32
<211>    30
<212>    DNA
<213>    Artificial Sequence

<220>
<223>    Aptamer


<400>    32
gcagcagggg gaggagcagc tcaggccttg                                        30


<210>    33
<211>    30
<212>    DNA
<213>    Artificial Sequence

<220>
<223>    Aptamer


<400>    33
gcagtaaggg aggagcgaat tacccaccgg                                        30

<210>	34
<211>	30
<212>	DNA
<213>	Artificial Sequence

<220>
<223>	Aptamer


<400>	34
gcaggtaacg agcacggggt cacgggtatg	30


<210>	35
<211>	30
<212>	DNA
<213>	Artificial Sequence

<220>
<223>	Aptamer


<400>	35
gcagcagagg gggagcagga cgactagtcg	30


<210>	36
<211>	30
<212>	DNA
<213>	Artificial Sequence

<220>
<223>	Aptamer


<400>	36
gcaggggacc gagcacgggg acgttcgagg	30


<210>	37
<211>	30
<212>	DNA
<213>	Artificial Sequence

<220>
<223>	Aptamer


<400>	37
gcagcaaggg gggagctaga gcatctcgtg	30


<210>	38
<211>	30
<212>	DNA
<213>	Artificial Sequence

<220>
<223>	Aptamer


<400>	38

ggacggagga gcacggagga gccgcgaatg                                    30

<210>    39
<211>    30
<212>    DNA
<213>    Artificial Sequence

<220>
<223>    Aptamer

<400>    39
ggcagcagag gggacgctgg ccaactctcg                                    30

<210>    40
<211>    30
<212>    DNA
<213>    Artificial Sequence

<220>
<223>    Aptamer

<400>    40
gcagcaaagg gaggagctga agtcacgttg                                    30

<210>    41
<211>    30
<212>    DNA
<213>    Artificial Sequence

<220>
<223>    Aptamer

<400>    41
gcagcaggga ggagctggtg accgtaccgg                                    30

<210>    42
<211>    30
<212>    DNA
<213>    Artificial Sequence

<220>
<223>    Aptamer

<400>    42
gcacgaaggg cagcaagggg gcagctgatg                                    30

<210>    43
<211>    30
<212>    DNA
<213>    Artificial Sequence

<220>
<223>    Aptamer

<400>    43
gcaggaggga ggagcaagga catgtccacg                                        30


<210>    44
<211>    30
<212>    DNA
<213>    Artificial Sequence

<220>
<223>    Aptamer


<400>    44
ggaccgaggg gagcacgggt ggagcacctg                                        30


<210>    45
<211>    30
<212>    DNA
<213>    Artificial Sequence

<220>
<223>    Aptamer


<400>    45
gcacgtcagg agcacgggga agcaagggtg                                        30


<210>    46
<211>    30
<212>    DNA
<213>    Artificial Sequence

<220>
<223>    Aptamer


<400>    46
gcacgaaagg agcacgtgga ggagcccgtg                                        30


<210>    47
<211>    20
<212>    DNA
<213>    Artificial Sequence

<220>
<223>    Aptamer


<400>    47
ggcagggagg agcaacgttg                                                   20


<210>    48
<211>    30
<212>    DNA
<213>    Artificial Sequence

<220>

<223>    Aptamer

<400>    48
gcagcaaggg ggagcaagaa ggtatccgtg                                        30

<210>    49
<211>    30
<212>    DNA
<213>    Artificial Sequence

<220>
<223>    Aptamer

<400>    49
ggaggggggg tgcggcgggg ccgtgtgtgg                                        30

<210>    50
<211>    29
<212>    DNA
<213>    Artificial Sequence

<220>
<223>    Aptamer

<400>    50
gcagcaggga ggagctcaga ggcctttgg                                         29

<210>    51
<211>    30
<212>    DNA
<213>    Artificial Sequence

<220>
<223>    Aptamer

<400>    51
ggacgtcagg agcacgggga cgagaatagg                                        30

<210>    52
<211>    30
<212>    DNA
<213>    Artificial Sequence

<220>
<223>    Aptamer

<400>    52
gcagcaaggg gacgtagaaa ggcactcgtg                                        30

<210>    53
<211>    30
<212>    DNA
<213>    Artificial Sequence

<220>
<223>    Aptamer


<400>    53
gcagcaaggg gggagcagag gacgctagtg                                        30


<210>    54
<211>    30
<212>    DNA
<213>    Artificial Sequence

<220>
<223>    Aptamer


<400>    54
gcagaaggga ggagcagatg agacggtacg                                        30


<210>    55
<211>    29
<212>    DNA
<213>    Artificial Sequence

<220>
<223>    Aptamer


<400>    55
gcagacaggg agacgcagct cgatactcg                                         29


<210>    56
<211>    30
<212>    DNA
<213>    Artificial Sequence

<220>
<223>    Aptamer


<400>    56
gcagcaaggg ggaagcagtg gagtacgttg                                        30


<210>    57
<211>    19
<212>    DNA
<213>    Artificial Sequence

<220>
<223>    Aptamer


<400>    57
gcagcaaggg gacggtggg                                                    19


<210>    58
<211>    21

<212>    DNA
<213>    Artificial Sequence

<220>
<223>    Aptamer


<400>    58
ggcagcaagg ggaagctcgt g                                                21


<210>    59
<211>    30
<212>    DNA
<213>    Artificial Sequence

<220>
<223>    Aptamer


<400>    59
gcagcaggga ggagcggtaa tagcatgtcg                                       30


<210>    60
<211>    30
<212>    DNA
<213>    Artificial Sequence

<220>
<223>    Aptamer


<400>    60
gcacgaagga gcacggggaa gccagccgtg                                       30


<210>    61
<211>    30
<212>    DNA
<213>    Artificial Sequence

<220>
<223>    Aptamer


<400>    61
gcagcaaggg ggacgtcgga tgaaccctgg                                       30


<210>    62
<211>    30
<212>    DNA
<213>    Artificial Sequence

<220>
<223>    Aptamer


<400>    62
ggccaaagca gcagggagga gcgatgattg                                       30

<210>	63
<211>	30
<212>	DNA
<213>	Artificial Sequence

<220>
<223>	Aptamer


<400>	63
gacgaaggag ctagcggggg agcaccccgg	30


<210>	64
<211>	30
<212>	DNA
<213>	Artificial Sequence

<220>
<223>	Aptamer


<400>	64
gcagcaaggg gacgtcagtg aagcctcatg	30


<210>	65
<211>	30
<212>	DNA
<213>	Artificial Sequence

<220>
<223>	Aptamer


<400>	65
gcagcaaggg gggagcagga ggcatgtgtg	30


<210>	66
<211>	29
<212>	DNA
<213>	Artificial Sequence

<220>
<223>	Aptamer


<400>	66
gcagtaaggg agaagcgttg gcctcgttg	29


<210>	67
<211>	30
<212>	DNA
<213>	Artificial Sequence

<220>
<223>	Aptamer


<400>	67
gcagtgaaac ggagtaaggg gccggacatg	30

<210>	68
<211>	30
<212>	DNA
<213>	Artificial Sequence

<220>
<223>	Aptamer


<400>	68
gcagtgttac gagcacgggg gagccttatg	30


<210>	69
<211>	30
<212>	DNA
<213>	Artificial Sequence

<220>
<223>	Aptamer


<400>	69
ggaggggggg gagcggcggg ggacgtcgtg	30


<210>	70
<211>	29
<212>	DNA
<213>	Artificial Sequence

<220>
<223>	Aptamer


<400>	70
gcagcaaggg ggacgtagac tcgtggttg	29


<210>	71
<211>	30
<212>	DNA
<213>	Artificial Sequence

<220>
<223>	Aptamer


<400>	71
gcagcaaagg ggacgctgat gcaaccctgg	30


<210>	72
<211>	30
<212>	DNA
<213>	Artificial Sequence

<220>
<223>	Aptamer

<400>    72
ggccagcaga ggggggagca gtgtgacgtg                                        30


<210>    73
<211>    30
<212>    DNA
<213>    Artificial Sequence

<220>
<223>    Aptamer


<400>    73
ccagcaaggg aggagcagct catggccacg                                        30


<210>    74
<211>    30
<212>    DNA
<213>    Artificial Sequence

<220>
<223>    Aptamer


<400>    74
gcacgaagtg ggaaggaggc cgcccggttg                                        30


<210>    75
<211>    30
<212>    DNA
<213>    Artificial Sequence

<220>
<223>    Aptamer


<400>    75
gcaggtacga gcacggggga agcgactcgg                                        30


<210>    76
<211>    30
<212>    DNA
<213>    Artificial Sequence

<220>
<223>    Aptamer


<400>    76
gcagcagaag aggagacgca gcaccgtgtg                                        30


<210>    77
<211>    30
<212>    DNA
<213>    Artificial Sequence

<220>
<223>    Aptamer

<400>    77
gacgaagaga gcgaaggggc cgcggttgtg                                        30


<210>    78
<211>    30
<212>    DNA
<213>    Artificial Sequence

<220>
<223>    Aptamer


<400>    78
gcagcagaag ggaggagcag tcgaacactg                                        30


<210>    79
<211>    30
<212>    DNA
<213>    Artificial Sequence

<220>
<223>    Aptamer


<400>    79
gcagcaaggg ggagcggtgg ccagttgtgg                                        30


<210>    80
<211>    30
<212>    DNA
<213>    Artificial Sequence

<220>
<223>    Aptamer


<400>    80
ggccaaagcg aagggagacg cggtttgtgg                                        30


<210>    81
<211>    20
<212>    DNA
<213>    Artificial Sequence

<220>
<223>    Aptamer


<400>    81
gcagcaaggg ggacgtgtgg                                                   20


<210>    82
<211>    30
<212>    DNA
<213>    Artificial Sequence

<220>
<223>    Aptamer

<400>    82
ggagggaagg gaggagcagg actgccctgg                                        30

<210>    83
<211>    30
<212>    DNA
<213>    Artificial Sequence

<220>
<223>    Aptamer

<400>    83
ggagagcaac ggagcagggg ccgcggtgtg                                        30

<210>    84
<211>    30
<212>    DNA
<213>    Artificial Sequence

<220>
<223>    Aptamer

<400>    84
ggacggagga caagcagggg ggccgccgtg                                        30

<210>    85
<211>    30
<212>    DNA
<213>    Artificial Sequence

<220>
<223>    Aptamer

<400>    85
gcacgaacgg agcacgggga cgtctgtcgg                                        30

<210>    86
<211>    30
<212>    DNA
<213>    Artificial Sequence

<220>
<223>    Aptamer

<400>    86
gcagcaaggg gaggagcagc taacgtgtgg                                        30

<210>    87
<211>    30
<212>    DNA

<213>    Artificial Sequence

<220>
<223>    Aptamer


<400>    87
gcacgaaagg gaggagctac agctgccatg                                        30


<210>    88
<211>    30
<212>    DNA
<213>    Artificial Sequence

<220>
<223>    Aptamer


<400>    88
gcacggaggg agcaaggggc cgccggctgg                                        30


<210>    89
<211>    30
<212>    DNA
<213>    Artificial Sequence

<220>
<223>    Aptamer


<400>    89
caacgaagtg gggaaggggc cgcggactgg                                        30


<210>    90
<211>    30
<212>    DNA
<213>    Artificial Sequence

<220>
<223>    Aptamer


<400>    90
gcacaaaggt gggaggggcc gcttctcctg                                        30


<210>    91
<211>    30
<212>    DNA
<213>    Artificial Sequence

<220>
<223>    Aptamer


<400>    91
gcagcaggga ggagcaagga tcatccgttg                                        30


<210>    92

<211>    29
<212>    DNA
<213>    Artificial Sequence

<220>
<223>    Aptamer


<400>    92
gcagggcaac gagcacgggg acgacggag                                          29


<210>    93
<211>    30
<212>    DNA
<213>    Artificial Sequence

<220>
<223>    Aptamer


<400>    93
gcagtgatca aacgagcacg gggccagtgg                                         30


<210>    94
<211>    30
<212>    DNA
<213>    Artificial Sequence

<220>
<223>    Aptamer


<400>    94
ggagtggtgc gagcacggga ggagcgtacg                                         30


<210>    95
<211>    30
<212>    DNA
<213>    Artificial Sequence

<220>
<223>    Aptamer


<400>    95
gcagcaaggg ggacgggact gagtgtatgg                                          30


<210>    96
<211>    30
<212>    DNA
<213>    Artificial Sequence

<220>
<223>    Aptamer


<400>    96
gcagcaggga ggagcggaat ggcaatgttg                                         30

<210>    97
<211>    30
<212>    DNA
<213>    Artificial Sequence

<220>
<223>    Aptamer


<400>    97
gcagcaaggg gccggggcga gacgtagttg                                          30


<210>    98
<211>    20
<212>    DNA
<213>    Artificial Sequence

<220>
<223>    Aptamer


<400>    98
ggagggagga gcgtgtgttg                                                    20


<210>    99
<211>    30
<212>    DNA
<213>    Artificial Sequence

<220>
<223>    Aptamer


<400>    99
gcacgttagg gcggtagggg ccgcggctgg                                         30


<210>    100
<211>    20
<212>    DNA
<213>    Artificial Sequence

<220>
<223>    Aptamer


<400>    100
gcagcaaggg gaacgtgtcg                                                    20


<210>    101
<211>    10
<212>    DNA
<213>    Artificial Sequence

<220>
<223>    Aptamer


<400>    101

gagcacgggg                                                              10


<210>    102
<211>    30
<212>    DNA
<213>    Artificial Sequence

<220>
<223>    Negative control


<400>    102
cgcgcgtcag gcattcctca caattcttcg                                        30


<210>    103
<211>    60
<212>    DNA
<213>    Artificial Sequence

<220>
<223>    Primer


<400>    103
atgcggatcc cgcgcnnnnn nnnnnnnnnn nnnnnnnnnn nnnnngcgcg aagcttgcgc      60

                                                                        60


<210>    104
<211>    15
<212>    DNA
<213>    Artificial Sequence

<220>
<223>    Primer


<400>    104
atgcggatcc cgcgc                                                        15


<210>    105
<211>    15
<212>    DNA
<213>    Artificial Sequence

<220>
<223>    Primer


<400>    105
gcgcaagctt cgcgc                                                        15